# EUROPÄISCHE PATENTSCHRIFT

(11) **EP 3 397 211 B1**
(45) Veröffentlichungstag und Bekanntmachung des Hinweises auf die Patenterteilung: **18.12.2019**
(21) Anmeldenummer: 16836224.2
(22) Anmeldetag: 28.12.2016
(51) Int. Cl.: A61F 2/36, B23K 1/00, B23K 1/19, B23K 35/26, B23K 35/32, B23K 35/24, A61F 2/30, B23K 103/14, B23K 103/00

(54) **ORTHOPÄDISCHES IMPLANTAT**
ORTHOPAEDIC IMPLANT
IMPLANT ORTHOPÉDIQUE

(30) Priorität: 29.12.2015 DE 102015016893
(43) Veröffentlichungstag der Anmeldung: 07.11.2018
(73) Patentinhaber: Mitrovic, Milija, 18055 Rostock (DE)
(72) Erfinder: Mitrovic, Milija, 18055 Rostock (DE)
(86) Internationale Anmeldenummer: PCT/DE2016/000443
(87) Internationale Veröffentlichungsnummer: WO 2017/114521

(56) Entgegenhaltungen:
- DE-A1- 10 340 059
- DE-A1- 19 952 918
- DE-A1-102012 014 345
- DE-T2- 69 016 110

## Beschreibung

Die Erfindung betrifft ein orthopädisches Implantat in Form einer femoralen Komponente einer Hüftgelenk-Endoprothese mit einem aus einem keramischen Werkstoff bestehenden Kopf, der auf einen Verankerungsschaft aufgesetzt ist, der seinerseits in einen Knochen einführbar und in diesem verankerbar ist, wobei der Kopf eine in etwa zylinderförmige sacklochartige innere Ausnehmung aufweist und der Verankerungsschaft mit einem Zapfen zum Einstecken in diese Ausnehmung versehen ist und wobei in die Ausnehmung des Kopfes eine ebenfalls in etwa zylinderförmige metallische Hülse eingelötet ist, über die der Keramikkopf durch ein Glaslot mit dem Zapfen verbindbar ist und wobei die Verbindung zwischen dem Keramikkopf und der Hülse über ein Gaslot hergestellt wird.

Die femorale Komponente einer Hüftgelenk-Endoprothese besteht im wesentlichen aus einem Schaft zur Verankerung im Kern des Röhrenknochens des Oberschenkels und einem angenähert kugelkalottenartigen Kopf zum Ersatz eines erkrankten Hüftkopfes, der am proximalen Ende des Verankerungsschaftes so angebracht ist, daß er mit dem Verankerungsschaft eine tragfähige Einheit bildet. Während bei derartigen Endoprothesen der Verankerungsschaft und der Kugelkopf häufig aus artgleichem Material bestehen und untrennbar miteinander verschweißt werden, ist bei einer anderen Verbindung zwischen dem Prothesenkopf und dem Verankerungsschaft eine Art Steckverbindung vorgesehen. Bei dieser ist ein tragender Zapfen in Form eines Konus am proximalen Ende des Verankerungsschaftes angeordnet, auf den wiederum der Prothesenkopf aufgesteckt ist. Eine solche Anordnung ist aus der DE 2921 529 A1 bekannt geworden. Die Verbindung zwischen dem Kopf und dem diesen tragenden Zapfen ist bei dieser bekannten Anordnung lösbar ausgebildet, wodurch es möglich ist, während der Operation je nach Indikation auf den tragenden Zapfen entweder einen Kopf mit größerem Durchmesser zum Ersatz eines erkrankten Hüftkopfes zu verwenden und dadurch eine sogenannte Hemiprothese zu implantieren, oder aber durch einen Kopf kleineren Durchmessers diesen mittels einer Ersatzpfanne zu einer Total-Hüftendoprothese zu vervollständigen.

Solche Hüftgelenk-Endoprothesen sind nur dann zuverlässig einsetzbar, wenn neben einer einwandfreien Verankerung des Prothesenschaftes im Femur auch die Langzeitfunktionen des den Kopf tragenden Zapfenlagers und des Kopfes selbst gewährleistet sind. So besteht insbesondere bei den für Prothesenköpfe aus Oxidkeramik üblicherweise verwendeten konischen Steckverbindungen zur Herstellung einer mechanisch festen und schlupffreien Verklemmung zwischen dem konusförmigen, in der Regel metallischen Lagerzapfen des Verankerungsschaftes und dem Metall- oder Keramikkopf das Problem der Auflösung einer ursprünglich mechanisch sicheren Verbindung sowie der Zerstörung der Oberfläche der Werkstoffpaarung aus Kopf und Schaft. Dadurch aber können Korrosionsvorgänge ausgelöst werden, die bisweilen die Integrität der Prothese insgesamt infrage stellen. Außerdem kann eine unzureichende Passung eines Keramikkopfes mit dem metallischen Zapfen zu erhöhten Spannungen in der Keramik mit Folge eines anschließenden Bruches der Implantatkomponente führen. Des Weiteren stellt das Aufstecken eines Keramikkopfes auf einen in-situ belassenen Schaft im Rahmen einer Wechseloperation immer einen kritischen Vorgang dar.

Aus diesem Grund ist es eine bereits bekannte Maßnahme, in einem Keramikkopf einer Hüftgelenk-Endoprothese eine Metallhülse vorzusehen, die einerseits mit dem Kugelkopf und andererseits mit dem Zapfen gefügt wird. Das Fügen der Hülse mit dem Kugelkopf geschieht bei den bislang bekannten Anordnungen entweder intraoperativ durch den Operateur oder aber sie wird bereits präoperativ in den Kugelkopf eingepresst. In diesem Zusammenhang ist aus der US 2006/0188845 A1 eine Anordnung bekannt, bei der eine metallische Hülse in eine ebenfalls metallische sacklochartige innere Ausnehmung eines Keramikkopfes wahlweise eingelötet oder eingeschrumpft wird.

Darüber hinaus ist aus der DE 102012014345 A1 eine femorale Komponente einer Hüftgelenk-Endoprothese der eingangs genannten Art bekannt geworden, bei der das Fügen von metallischer Hülse mit der sacklochartigen inneren Ausnehmung eines Keramikkopfes in jedem Fall präoperativ erfolgt, wobei die Verbindung zwischen dem Keramikkopf und der Hülse über ein Gaslot hergestellt wird.

Aufgabe der Erfindung ist es, ein solches Implantat derart auszubilden, dass der Keramikkopf und der Verankerungsschaft präoperativ derart miteinander verbunden sind, dass eine feste, nicht lösbare Verbindung zwischen der Hülse und dem Kopf entsteht und dass dadurch unter anderem kein Abrieb der artikulierenden Materialien von Kopf und Schaft freigesetzt werden kann.

Die Erfindung löst diese Aufgabe dadurch, dass die die Deckfläche der Hülse eine in etwa zentrische Durchgangsbohrung aufweist, durch die überschüssiges flüssiges zweites Glaslot in einen zwischen der und der Auflage des Schaftes bestehenden Hohlraum entweichen kann.

Indem femer bei einer bevorzugten Ausführungsform der Erfindung die Hülse und der Verankerungsschaft aus dem gleichen metallischen Material bestehen, wobei es sich vorzugweise um eine hochfeste Titanlegierung, in der Regel um die Legierung Ti-6Al-4V (Titanium Grade 5), handelt, wird eine besonders optimale Ausbildung des erfindungsgemäßen Implantates erreicht.

Nachfolgend soll die Erfindung anhand eines in der Zeichnung dargestellten Ausführungsbeispiels näher erläutert werden. Es zeigen:
- Fig. 1: eine Hüftgelenk-Endoprothese in perspektivischer Darstellung,
- Fig.2: eine Explosionsdarstellung der Anordnung gemäß Fig. 1,
- Fig.3: einen vergrößerten Schnitt durch den Kugelkopf der Hüftgelenk-Endoprothese gemäß Fig. 1 und
- Fig.4: einen vergrößerten Schnitt durch einen alternativen Kugelkopf einer Hüftgelenk-Endoprothese in drei unterschiedlichen Darstellungen.

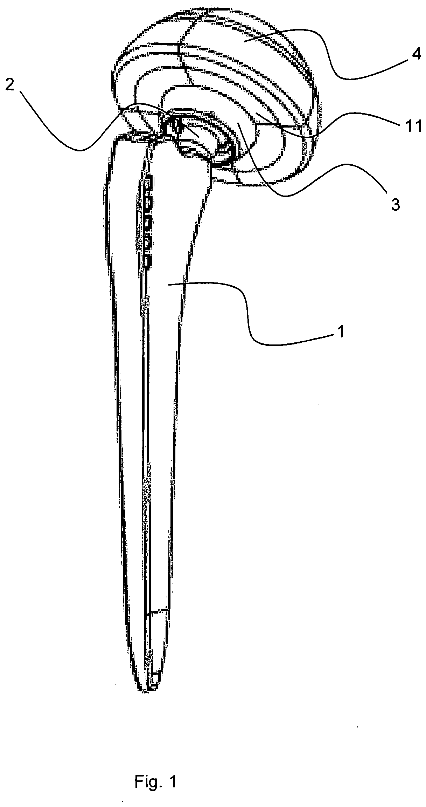

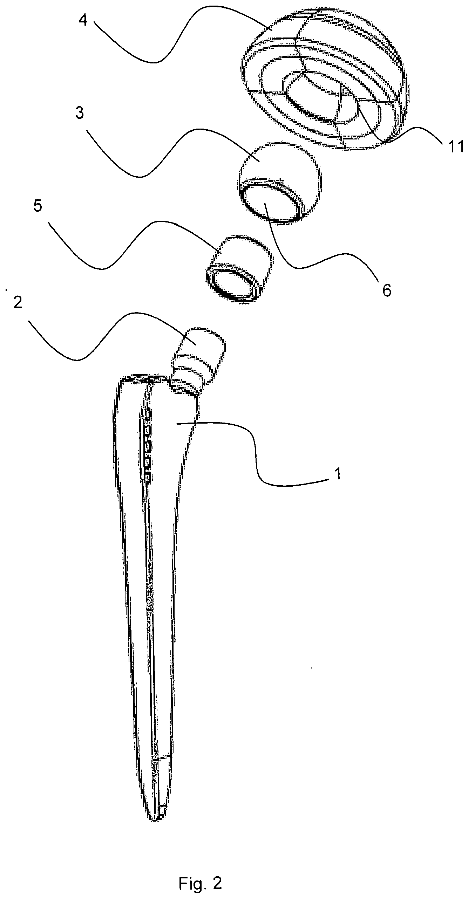

Die Darstellung gemäß der Figuren 1 und 2 zeigt eine femorale Komponente einer Hüftgelenk-Endoprothese mit einem Schaft 1 zur Verankerung im Kern des Röhrenknochen des Oberschenkels eines Patienten sowie mit einem auf eine proximale Auflage 2 des Schaftes 1 aufsetzbaren, in etwa kugelkalottenartig ausgebildeten Kopf 3 zum Ersatz des erkrankten Hüftkopfes eines Patienten. Dieser Kopf 3 ist in die Ersatzpfanne 4 einer Total-Hüftendoprothese eingesetzt, wobei ein zusätzlicher Pfanneneinsatz 11 in der Ersatzpfanne 4 gegebenenfalls eine perfekte Passung der beiden Komponenten aufeinander bewirkt.

Der Kopf 3 besteht aus einer Oxidkeramik und weist eine sacklochartige Ausnehmung 6 auf, in die eine Hülse 5 eingelötet ist, die aus dem gleichen Werkstoff besteht, aus dem auch der

Schaft 1 sowie die proximale Auflage 2 des Schaftes 1 bestehen. Im Fall des hier beschriebenen Ausführungsbeispiel handelt es sich dabei um die hochfeste Titanlegierung Ti-6Al-4V (Titanium Grade 5). Die Deckfläche der Hülse 5 ist mit einer Durchgangsbohrung 7 versehen. Auf die Außenfläche der Titanhülse 5 ist mittels Airbrush-Technik ein silikatisches Glaslot (8) aufgebracht, das unter anderem eine gleichmäßige Abdeckung und Bindung der Titanoxide vor dem Löten mittels eines Brennvorganges bewirkt. Diese Abdeckung gewährleistet ein homogenes Fliessverhalten eines anschließend aufgebrachten silikatischen Glaslotes 9. Die Durchgangsbohrung 7 übt dabei eine Kapillarwirkung auf das flüssige Glaslot 9 aus. Ziel dieser Aktion ist es, ein Auftreten oxidischer Produkte des Titans und eine möglicherweise damit einhergehende Tröpfchenbildung im Bereich der Hülse 5 zu verhindern, weil dies ansonsten zu einem Brechen der Hülse 5 führen könnte.

Beim anschließenden Brennvorgang wird der Keramikkopf 3 durch das Glaslot 9 mit der Titanhülse 5 verbunden, wobei diese Verbindung über das sich im keramischen Brand verfestigende silikatische Glaslot 8 hergestellt wird. Während die Außenfläche des Keramikkopfes 3 bei diesem Arbeitsgang nicht modifiziert wird, sind seine mit dem Glaslot 9 behandelten Areale der inneren Oberfläche mit Ausnahme des mit der Durchgangsbohrung 7 versehenen Bereiches der Deckfläche derTitanhülse 5 von dieser Hülse 5 überdeckt, so dass praktisch keine freie, mit der Umgebung interagierende modifizierte Oberfläche besteht. Während das überschüssige flüssige Glaslot 9 in den zwischen der Hülse 5 und der Auflage 2 des Schaftes 1 gebildeten Hohlraum 10 entweichen kann, bildet der beim Brennvorgang mittels Löten verfestigte Teil des Glaslotes 9 eine keramische festsitzende Schicht zwischen der inneren Oberfläche des Keramikkopfes 3 und der Außenfläche der Titanhülse 5 und bindet diese beide Komponenten fest aneinander.

Durch die auf diese Weise eingelötete Titanhülse 5 werden kritische Spannungen im Keramikkopf 3, die ansonsten beim Fügevorgang des Kopfes 3 auf den metallischen Zapfen 2 auftreten könnten, zuverlässig vermieden. Der keramische Kugelkopf 3 artikuliert mit der künstlichen Pfanne 4 bzw. mit dem Pfanneneinsatz 11 ; ein Kontakt des Kugelkopfes 3 zum umgebenden Knochen besteht nicht.

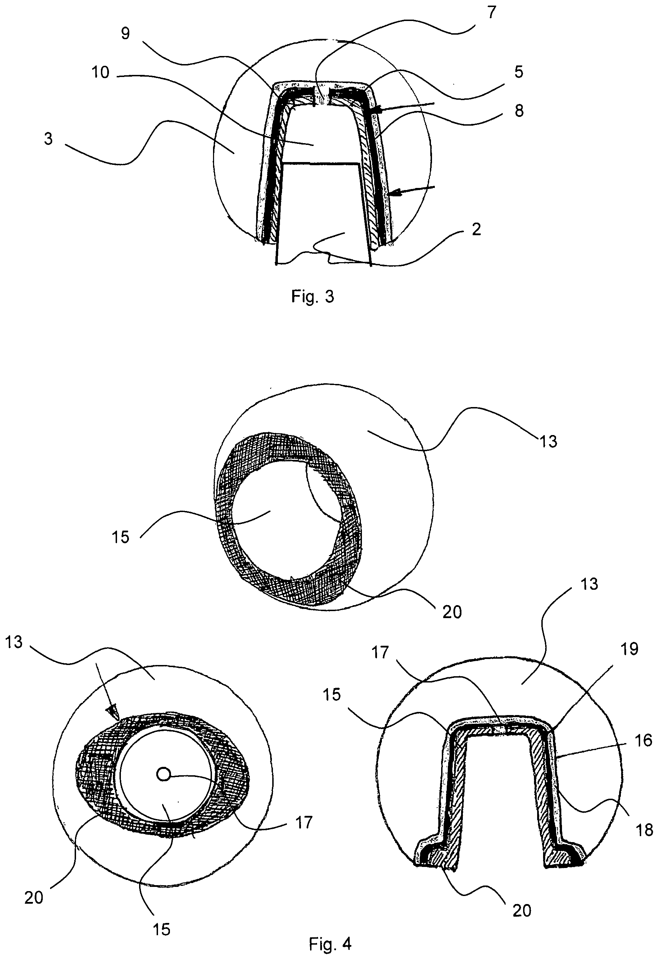

Die Abbildungen der Fig. 4 zeigen im Prinzip den gleichen Keramikkopf 13, wie er auch in den vorangehenden Darstellungen gezeigt ist, und auch der Ablauf des

Brennvorganges mittels Löten zwischen dem Keramikkopf 13 und einer in diesen eingesetzten Hülse 15 aus Ti-6Al-4V ist der gleiche wie vorangehend beschrieben, nur ist in diesem Fall der untere, verbreiterte Rand 20 der Hülse 15 elliptisch ausgebildet. In dieser Form dient er als zusätzlicher Verdrehschutz für die in den Kugelkopf 13 eingelötete Hülse 15 und verhindert in erster Linie bei Drehbewegungen des Kugelkopfes 13 das Einwirken von Torsionskräften auf das verfestigte Glaslot 19 zwischen der Außenseite der Hülse 15 und der Ausnehmung 16 des Kugelkopfes 13, während auch in diesen Fall überschüssiges flüssiges Glaslot 19 wieder über eine zentrische Durchgangsbohrung 17 in den zwischen dem oberen Teil der Hülse 15 und den darein ragenden oberen Endbereich der Auflage des Schaftes gebildeten Hohlraum abfließen kann.

## Patentansprüche

1. Orthopädisches Implantat in Form einer femoralen Komponente einer Hüftgelenk-Endoprothese mit einem aus einem keramischen Werkstoff bestehenden Kopf (3), derauf einen Verankerungsschaft (1) aufgesetzt ist, der seinerseits in einen Knochen einführbar und in diesem verankerbar ist, wobei der Keramikkopf (3) eine in etwa zylinderförmige sacklochartige innere Ausnehmung aufweist und der Verankerungsschaft (1) mit einem Zapfen zum Einstecken in diese Ausnehmung versehen ist und wobei in die Ausnehmung des Kopfes eine ebenfalls in etwa zylinderförmige metallische Hülse (5) eingelötet ist, über die der Keramikkopf (3) mit dem Zapfen verbindbar ist, und wobei die Verbindung zwischen dem Keramikkopf (3) und der Hülse (5) über ein Gaslot hergestellt wird, wobei die Hülse (5) durch ein in einem keramischen Brand sich verfestigendes silikatisches Glaslot (9) vorbeschichtet ist und dass die Verbindung zwischen dem Keramikkopf (3) und der Hülse (5) über ein zweites silikatisches Glaslot (8) hergestellt wird, **dadurch gekennzeichnet, dass** die Deckfläche der Hülse (5) eine in etwa zentrische Durchgangsbohrung (7) aufweist, durch die überschüssiges flüssiges zweites Glaslot (9) in einen zwischen der Hülse (5) und der Auflage (2) des Schaftes (1) bestehenden Hohlraum (10) entweichen kann.

2. Implantat nach Anspruch 1 , **dadurch gekennzeichnet, dass** der Kopf (3) aus einer Keramik auf der Basis von Zirkoniumdioxid besteht.

3. Implantat nach Anspruch 1 , **dadurch gekennzeichnet, dass** der Kopf (3) aus einer Keramik auf der Basis von Aluminiumoxid besteht.

4. Implantat nach Anspruch 1 , **dadurch gekennzeichnet, dass** der Kopf (3) aus einer Keramik auf der Basis von Mischkeramik besteht.

5. Implantat nach einem der Ansprüche 1 bis 4, **dadurch gekennzeichnet, dass** der Verankerungsschaft (1), der Zapfen (2) und die Hülse (5) aus dem gleichen metallischen Werkstoff bestehen.

6. Implantat nach einem der Ansprüche 1 bis 4, **dadurch gekennzeichnet, dass** der Verankerungsschaft (1), der Zapfen (2) und die Hülse (5) aus der hochfesten Titanlegierung Ti-6Al-4V bestehen.

## Claims

1. An orthopaedic implant in the form of a femoral component of a hip joint endoprosthesis with a head (3) made from a ceramic material which is mounted on an anchoring shaft (1), which in turn is insertable in a bone and can be anchored therein, wherein the ceramic head (3) has an approximately cylindrical inner recess in the form of a blind hole, and the anchoring shaft (1) is furnished with a pin for insertion in said recess, and wherein a metallic sleeve (5) which is also approximately cylindrical is soldered into the recess of the head, via which sleeve the ceramic head (3) can be connected to the pin, and wherein the connection between the ceramic head (3) and the sleeve (5) is made by means of a glass solder, wherein the sleeve (5) is pre-coated with a silicious glass solder (9) which is strengthened in a ceramic firing and that the connection between the ceramic head (3) and the sleeve (5) is created by a second silicious glass solder (8), **characterized in .that** the covering surface of the sleeve (5) has an approximately centrally arranged through-hole (7) through which the excess second glass solder (9) in liquid form can escape into a cavity (10) that exists between the sleeve (5) and the support (2) of the shaft (1).

2. The implant according to Claim 1, **characterized in that** the head (3) is made from a ceramic based on zirconium dioxide.

3. The implant according to Claim 1, **characterized in that** the head (3) is made from a ceramic based on aluminium dioxide.

4. The implant according to Claim 1, **characterized in that** the head (3) is made from a ceramic based on mixed ceramic.

5. The implant according to any one of Claims 1 to 4, **characterized in that** the anchoring shaft (1), the pin (2) and the sleeve (5) are made from the same metallic material.

6. The implant according to any one of Claims 1 to 4, **characterized in that** the anchoring shaft (1), the pin (2) and the sleeve (5) are made from the high-strength titanium alloy Ti-6Al-4V.

## Revendications

1. Implant orthopédique sous la forme d'un composant fémoral d'une endoprothèse pour l'articulation de la hanche, pourvu d'une tête (3) en une matière céramique, qui est posée sur une tige d'ancrage (1) qui pour sa part est susceptible d'être introduite dans un os et ancrée dans celui-ci, la tête en céramique (3) comportant un évidement interne en forme d'un trou borgne approximativement cylindrique et la tige d'ancrage (1) étant munie d'un tourillon destiné à être inséré dans ledit évidement et dans l'évidement de la tête étant brasée une douille (5) métallique, également de forme approximativement cylindrique, par l'intermédiaire de laquelle la tête en céramique (3) est susceptible d'être assemblée avec le tourillon, et l'assemblage entre la tête en céramique (3) et la douille (5) étant créée par brasure de verre, la douille (5) étant préalablement revêtue d'une brasure de verre (9) silicatée qui se solidifie lors de la cuisson de la céramique et l'assemblage entre la tête en céramique (3) et la douille (5) étant créé par l'intermédiaire d'une deuxième brasure de verre (8), **caractérisé en ce que** la surface de recouvrement de la douille (5) comporte un perçage traversant (7) approximativement centré, à travers lequel de la deuxième brasure de verre (9) liquide excédentaire peut s'échapper dans une cavité creuse (10) restant entre la douille (5) et l'habillage (2) de la tige (1).

2. Implant selon la revendication 1, **caractérisé en ce que** la tête (3) est constituée d'une céramique sur la base de dioxyde de zirconium.

3. Implant selon la revendication 1, **caractérisé en ce que** la tête (3) est constituée d'une céramique sur la base d'oxyde d'aluminium.

4. Implant selon la revendication 1, **caractérisé en ce que** la tête (3) est constituée d'une céramique sur la base de céramique mixte.

5. Implant selon l'une quelconque des revendications 1 à 4, **caractérisé en ce que** la tige d'ancrage (1), le tourillon (2) et la douille (5) sont constitués de la même matière métallique.

6. Implant selon l'une quelconque des revendications 1 à 4, **caractérisé en ce que** la tige d'ancrage (1), le tourillon (2) et la douille (5) sont constitués d'un alliage de titane ultrasolide en Ti-6Al-4V.
